# EUROPEAN PATENT SPECIFICATION

(11) **EP 3 270 942 B1**
(45) Date of publication and mention of the grant of the patent: **04.08.2021**
(21) Application number: 16764347.7
(22) Date of filing: 17.03.2016
(51) Int. Cl.: A61Q 1/02, A61Q 1/10, A61Q 1/12, A61Q 19/08, A61K 8/9789, A61K 36/33

(54) **USE OF HYLOCEREUS UNDATUS FRUIT EXTRACT AS FLUORESCENT COLORANT OF SKIN**
VERWENDUNG VON HYLOCEREUS-UNDATUS-FRUCHTEXTRAKT ALS FLUORESZIERENDER HAUTFARBSTOFF
UTILISATION D'EXTRAIT DE FRUIT D'HYLOCEREUS UNDATUS COMME COLORANT FLUORESCENT DE PEAU

(30) Priority: 19.03.2015 US 201562135193 P
(43) Date of publication of application: 24.01.2018
(73) Proprietor: I.B.R. Israeli Biotechnology Research LTD., 8122503 Yavne (IL)
(72) Inventor: VON OPPEN-BEZALEL, Liki, 14163 Berlin (DE); HAVAS, Fabien, 7936700 Bnei Dkalim (IL); KALO, Eyal, Ness- Ziona (IL); BEN-CHITRIT, Olga, Ashdod 7751101 (IL); PERRY, Inon, 6515012 Tel Aviv (IL)
(74) Representative: Trinks, Ole
(86) International application number: PCT/IL2016/050294
(87) International publication number: WO 2016/147189

(56) References cited:
- CN-A- 103 417 418
- US-A1- 2006 160 702
- US-A1- 2009 264 291
- DATABASE MEDLINE [Online] US NATIONAL LIBRARY OF MEDICINE (NLM), BETHESDA, MD, US; January 2010 (2010-01), ANAND SWARUP KOLLA R L ET AL: "Effect of dragon fruit extract on oxidative stress and aortic stiffness in streptozotocin-induced diabetes in rats.", XP002785543, Database accession no. NLM21808536
- JAYAKUMAR R ET AL: "Inhibitory effects of fruit extracts on nitric oxide-induced proliferation in MCF-7 cells", FOOD CHEMISTRY, ELSEVIER LTD, NL, vol. 126, no. 3, 1 June 2011 (2011-06-01), pages 956-960, XP027600993, ISSN: 0308-8146, DOI: 10.1016/J.FOODCHEM.2010.11.093 [retrieved on 2011-01-13]
- DATABASE GNPD [Online] MINTEL; 20 August 2013 (2013-08-20), anonymous: "Anti-Age Day Care SPF 15", XP055514112, retrieved from www.gnpd.com Database accession no. 2123985
- DATABASE GNPD [Online] MINTEL; 22 August 2014 (2014-08-22), anonymous: "Purifying Facial Scrub", XP055514122, retrieved from www.gnpd.com Database accession no. 2578125
- DATABASE GNPD [Online] MINTEL; 29 February 2012 (2012-02-29), anonymous: "Resurfacing Eye Serum", XP055514128, retrieved from www.gnpd.com Database accession no. 1746797
- DATABASE WPI Week 201413 Thomson Scientific, London, GB; AN 2014-C14078 XP002785565, & CN 103 417 418 A (SUN Y) 4 December 2013 (2013-12-04)

## Description

### FIELD OF THE INVENTION

The present invention relates to the use of *Hylocereus Undatus* fruit extract as light activated fluorescent colorant in cosmetic compositions. Cosmetic compositions include all skin products where an even, flawless and natural skin appearance is desired.

### BACKGROUND OF THE INVENTION

Standard definitions of beautiful skin include skin having uniform undertones of color. Skin color depends on the skin structure itself. The outer layer of human skin, the stratum corneum, is a semi-transparent layer that permits glimpses of the deeper layers of skin, where blood vessels and pigments reside. What is viewed as the skin's color results from a combination of the reddish hue of the blood vessels' hemoglobin, the brown/black hue of melanin, the primary skin pigment, and the reflection of light from the skin layers. Yellow undertones of the skin are the result of the presence of small amounts of brown melanin and presence of collagen. Smooth and even surface, lack of visible flaws and uniform color distribution are the most desirable elements of skin beauty and appearance.

Cosmetic products include products aiming at reducing the visual perception of skin imperfections. Preferred compounds are those that give the skin a natural look, while still providing coverage of flaws and unevenness of skin tone.

Fluorescent agents have been proposed to be used in cosmetic compositions. For example, U.S. Patent No. 6,613,359 discloses optically-activated particles for use in cosmetic preparations, toiletries or pharmaceutical preparations to reduce the visual perception of cellulite, shadows, skin discolorations and wrinkles.

U.S. Patent No. 6,313,181 discloses cosmetic compositions comprising a fluorescent-effective amount of at least one fluorescent brightener, in combination with a cosmetically acceptable vehicle. The compositions, when applied to the skin, replenish the fluorescence that may have been lost due to chrono- or photoaging, while not conferring any readily discernible amount of color on the skin.

U.S. Patent Application Publication No. 2006/0078518 discloses cosmetic or personal care compositions which comprise at least one fluorescent colorant other than white and at least one non-fluorescent colorant, useful in preparing colored cosmetic or personal care preparations providing an enhanced skin appearance. U.S. Patent Application Publication No. 2009/0288674 discloses cosmetic dye composition with a lightening effect for human keratin materials, comprising at least one fluorescent dye and at least one aminosilicone, and process of dyeing keratin fibers, particularly human keratin fiber.

US 2009/264291 A1 and US 2006/160702 A1 teach the use of a plant derived aqueous extract, including Hylocereus Undatus fruit extract, capable of inhibiting proliferation of exogenic cells and thus to slow the cell proliferation and the metabolic rate of cells or tissues, inhibiting thus the undesired or deleterious cell proliferation in plant or mammal tissue.

Hylocereus is a genus of cacti with about 16 species known in Central America and Mexico. The most known species are those having edible fruit, also called "pitaya" "pitahaya" and "dragon fruit", including Hylocereus polyrhizus (red dragon fruit), Hylocereus undatus (white dragon fruit) and Hylocereus megalanthus (yellow dragon fruit). Varieties of Hylocereus megalanthus, Hylocereus polyrhizus and Hylocereus undatus as well as hybrids of these three species are grown commercially worldwide. H. undatus has white pulp with pink skin, H. polyrhizus has red pulp with pink skin while H. megalanthus has white pulp with yellow skin. The pulp is delicate and juicy and contains numerous small soft seeds.

Dragon fruit are known to be rich in vitamins and antioxidants. Red dragon fruit (H. polyrhizus) are rich in betalain pigments including betacyanins and betaxanthins. Betalains are used as natural food colorants in various areas of the food industry.

The Applicant of the present invention has previously disclosed aqueous extract of white dragon fruit (H. undatus) having use in cosmetics, particularly for preventing skin aging by slowing down cell proliferation (U.S. Patent Application Publications No. 2006/0160702; 2009/0264291). The extract was also found to have impact on the intrinsic luminescence of the skin and to maintain skin hydration.

There is still a need for skin compatible compounds for cosmetic use, offering camouflage of visible flaws and uniformity of skin color.

### SUMMARY OF THE INVENTION

The present invention provides a use of an extract of Hylocereus undatus (white dragon) fruit as defined by the claims.

The present invention is based in part on the unexpected discovery that under sunlight and/or UV radiation, an extract of the H. undatus fruit fluoresce at the blue range wavelength. Such fluorescence is highly unexpected, because the fruit of H. undatus is white, and is not known to have significant amounts of pigments like the pigments betalains present in the red dragon fruit. Furthermore, betalains are known to emit fluorescent light at the green area, while the extract of the present invention shows significant emission in the blue range. Without wishing to be bound by any specific theory or mechanism of action, the fluorescence activity may be attributed to the extract preparation process.

The unexpected blue light emission of the H. undatus fruit extract under sun and/or UV light is highly advantageous in cosmetics, particularly for masking skin imperfection. According to the color opponent process, the human eye record differences between opponent channels (red versus green, blue versus yellow, and black versus white). Responses to one color of an opponent channel are antagonistic to those to the other color. Thus, blue hue on a yellowish skin would result in white perception to the eye, eliminating flaws from visibility.

In addition, the H. undatus fruit extract is essentially colorless, and thus its application on a subject skin preserves the natural appearance of the skin while masking spots.

According to one aspect, the present disclosure provides a method for reducing the visual perception of skin imperfection, the method comprises applying onto an imperfect skin area of a human subject in need thereof H. undatus fruit extract or a composition comprising same in an amount sufficient to provide thin layer cover of the imperfect skin area, thereby reducing the visual perception of imperfection of said skin area when exposed to a light source comprising ultraviolet (UV) light.

According to some embodiments, the light source comprising UV light is sunlight. According to certain embodiments, the method further comprises a step of identifying a human subject in need thereof. According to some embodiments, identifying a human subject in need thereof is based on information received from the subject indicating said subject need for reducing the visual perception of skin imperfection. According to some embodiments, the information is received from answers of the subject to a questionnaire. According to yet additional embodiments, identifying a human subject in need thereof is based on professional assessment of the subject skin, particularly facial skin.

According to certain embodiments, the skin undertone color of the human subject is selected from the group consisting of yellow, yellow to olive and olive undertone. Each possibility represents a separate embodiment of the present invention.

According to certain embodiments, the skin imperfection is selected from the group consisting of skin discolorations, open pores, mild scars, blotchiness and any combination thereof. Each possibility represents a separate embodiment of the present invention.

According to some embodiments, the method results in a visual perception of even skin tone.

The extract is obtained from the fleshy, edible pulp of the fruit. It is to be explicitly understood that the pericarp (known as skin, rind or peel) of the fruit does not constitute part of the extract.

According to certain embodiments, the H. undatus fruit extract is characterized by fluorescence emission at the blue range upon excitation over an extended wavelength range of from about 340 nm to about 395 nm.

According to certain exemplary embodiments, the H. undatus fruit extract is characterized by a fluorescence emission having a peak at a wavelength range of from 460 nm to 470 nm upon excitation with light having a wavelength of 370 nm to 390 nm.

The extract used as light activated fluorescent colorant is produced by a process comprising the steps of:
(a) separating the edible fruit flesh from the pericarp;
(b) milling the fruit flesh to obtain a slurry;
(c) separating insoluble solids from the slurry to obtain a pulp-free juice;
(d) heating the pulp-free juice; and
(e) separating insoluble solids from the heated pulp-free juice to obtain a liquid extract.

According to certain embodiments, the pulp free juice is heated at 100-120 °C.

Optionally, suitable preservative(s) and/or stabilizer(s) are added to the liquid extract.

According to certain embodiments, separating insoluble solids at steps (c) or (e) is performed by centrifugation, filtration or a combination thereof. Each possibility represents a separate embodiment of the present invention.

The H. undatus fruit extract of the present disclosure can be administered per se, or can be incorporated within a cosmetic composition.

Any cosmetic composition known to be suitable for topical application to the skin can be used according to the teachings of the present invention as long the formulation preserves the ability of the extract to fluoresce under UV and/or visible light.

According to certain embodiments, the H. undatus fruit extract is formulated in a cosmetic composition further comprising a cosmetically acceptable diluent, carrier or excipient.

According to certain embodiments, the cosmetic composition further comprises an additive including, but not limited to, stabilizers, preservatives, pigments, pH buffers, emollients, disinfectants, fats, emulsifiers and co-emulsifiers, hydrophilic or lipophilic gelling agents, solvents, fragrances, fillers, dyestuffs, neutralizers, penetration-enhancing agents and polymers and any combination thereof. The quantities of these various additives are those conventionally used in cosmetic and dermatological preparations as is known to a person skilled in the art. Each possibility represents a separate embodiment of the present invention.

According to certain exemplary embodiments, the cosmetic composition comprising H. undatus fruit extract and at least one stabilizer and/or preservative. According to some embodiments, the preservative and/or stabilizer is selected from the group consisting of sodium metabisulfite, sodium sulfite, phenoxyethanol, parabens, benzoic acid, sodium benzoate, benzyl alcohol and any combination thereof. As exemplified herein below, the composition comprising the preservative and/or stabilizer maintains its fluorescence characteristics.

According to certain exemplary embodiments, the cosmetic composition comprising H. undatus fruit extract and at least one solvent. According to certain embodiments, the additional solvent is selected from the group consisting of ethanol, propylene glycol, butylene glycol, methanol, glycerol and acetone. Each possibility represents a separate embodiment of the present invention. The cosmetic composition according to the present diisclosure may be further formulated for cosmetic use in a form selected from the group consisting of, ointments, creams, emulsions, suspensions, dispersions, powders or solutions.

According to certain exemplary embodiments, the cosmetic compositions of the present disclosure are formulated in a form selected from the group consisting of foundation, concealer, BB cream, CC cream, facial powder and body powder. Each possibility represents a separate embodiment of the present invention.

According to additional embodiments, the cosmetic compositions of the present disclosure are formulated in a form selected from the group consisting of blush, highlighter, eyeshadow, eyeliner and contouring preparations. Each possibility represents a separate embodiment of the present disclosure.

According to certain exemplary embodiments, the cosmetic composition of the present disclosure is in a form of facial foundation. The facial foundation can be in the form of liquid or powder (compressed or loose). According to other exemplary embodiments, the cosmetic composition of the present diisclosure is in a form of concealer. According to yet additional exemplary embodiments, the composition of the invention is in a form of facial or body powder.

According to certain exemplary embodiments, the cosmetic compositions and/or formulations comprising the H. undatus fruit extract are non-color cosmetics thereby maintaining a perception of vivacious look of clean bare skin. According to other embodiments, the cosmetic compositions and/or formulations further comprise cosmetically acceptable colorant. According to certain exemplary embodiments, the at least one colorant is selected from the group consisting of inorganic pigments, natural colorants, synthetic organic monomeric colorants, synthetic organic polymeric colorants, a self-tanner, and combinations thereof. Each possibility represents a separate embodiment of the present disclosure.

According to certain embodiments, the cosmetic composition and/or formulation comprises from about 0.1 % to about 100% of the H. undatus fruit extract. According to other embodiments, the cosmetic composition and/or formulation comprises the H. undatus fruit extract in a concentration of from about 0.5% to about 50%. According to certain currently exemplary embodiments, the concentration of the H. undatus fruit extract is from about 0.5% to about 5%. According to certain exemplary embodiments, the cosmetic composition and/or formulation comprises the H. undatus fruit extract in a concentration of 1 %.

According to certain exemplary embodiments, the method of the present disclosure comprises applying onto an imperfect skin area of a subject a cosmetic composition comprising from 1 % to 5% H. undatus fruit extract. According to further exemplary embodiments, the composition comprises 2% H. undatus fruit extract. According to additional exemplary embodiments, the composition comprises 1 % H. undatus fruit extract. According to some embodiments, the amount of the cosmetic composition applied onto the imperfect skin area is from about 0.1 g to about 5.0 g. According to certain exemplary embodiments, the amount applied is from about 0.5 g to about 1.0 g.

It is to be explicitly understood that reduction in the visual perception of skin imperfection by the extract of the invention or a cosmetic composition comprising same lasts as long as the skin area is covered.

According to certain embodiments, the reduction in the visual perception of skin imperfection lasts for up to 12 hours. According to other embodiments, the reduction in the visual perception of skin imperfection lasts for up to 8 hours According to additional embodiments, the reduction in the visual perception of skin imperfection lasts for 2-4 hours.

Other objects, features and advantages of the present disclosure will become clear from the following description and drawings.

### BRIEF DESCRIPTION OF THE FIGURES

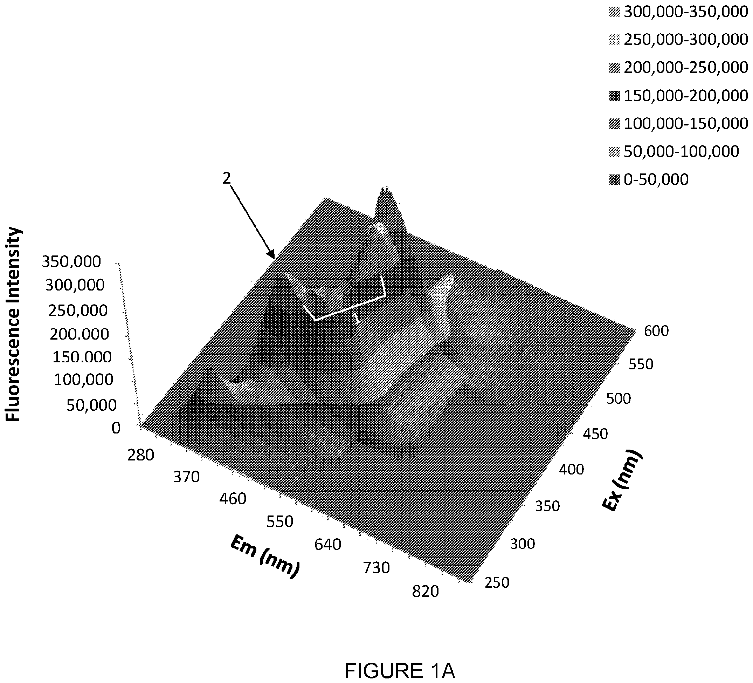

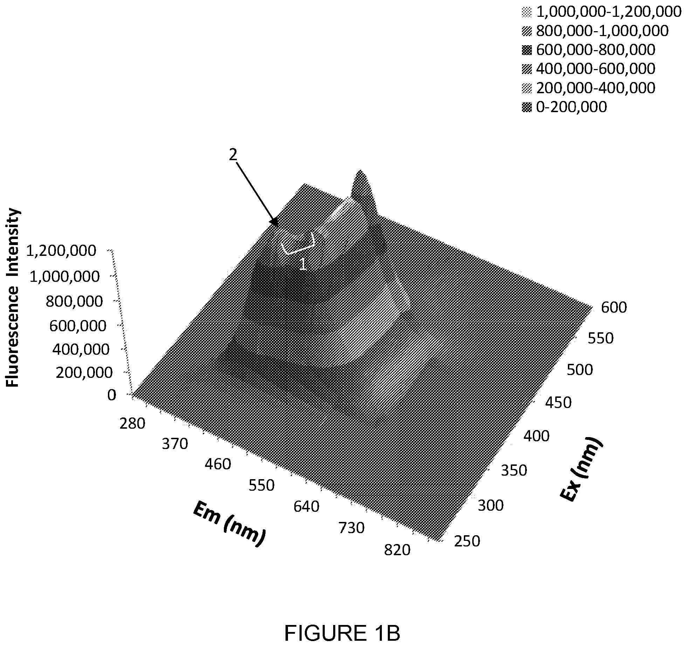

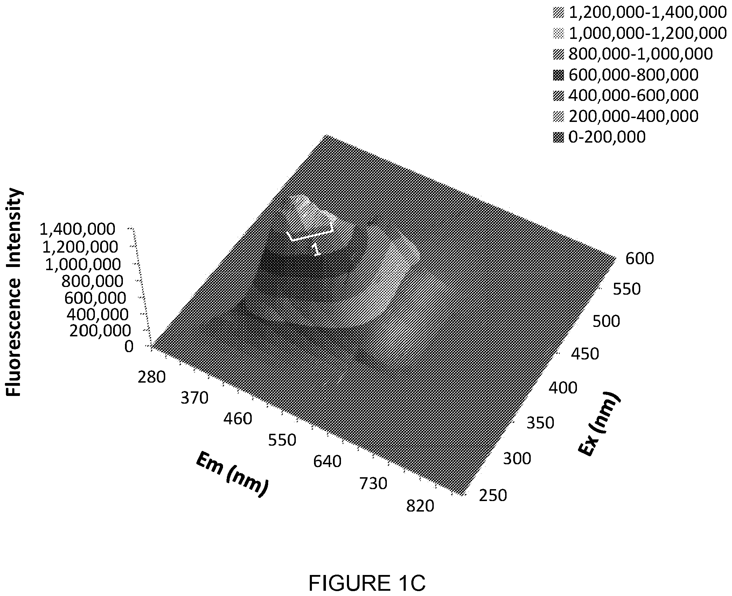

FIG. 1 shows fluorescence of the H. undatus fruit extract of the invention with and without a stabilizer and a preservative compared to a H. undatus pulp-free juice. Fig. 1A: Fluorescence of H. undatus pulp-free juice (1) Ex-390-370; Em-463-466 (Bright Blue); (2) Ex-340-360; Em- 433-439 (Blue). Fig. IB: Fluorescence of H. undatus fruit extract (without preservatives). (1) Ex-390-370; Em-463-466 (Bright Blue); (2) Ex-340-360; Em- 433-439 (Blue). Fig. 1C: Fluorescence of H. undatus fruit extract containing the preservatives phenoxyethanol and the stabilizer sodium metabisulfite. (1) Ex- 360-390; Em- 445-463 (Blue).

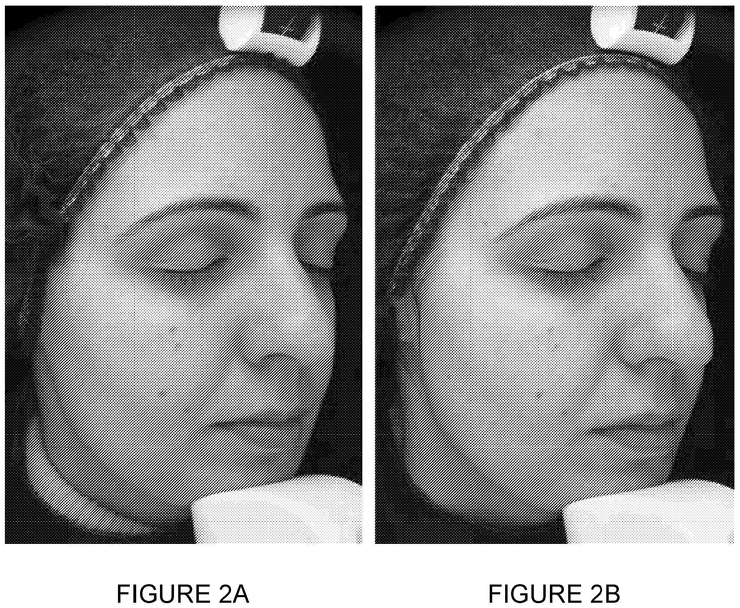

FIG. 2 demonstrates facial skin exposed to full spectrum light. Figure 2A shows a picture of bare face and Figure 2B shows a picture of the face to which a cream containing 1 % of H. undatus fruit extract was applied.

### DETAILED DESCRIPTION OF THE INVENTION

The present invention provides a use of Hylocereus Undatus fruit extract as light activated fluorescent colorant of skin for reducing the visual perception of skin imperfection as defined by independent claim 1, wherein further developments of the inventive use are provided in the sub-claims, respectively.

### Definitions

As used herein, the terms "extract of Hylocereus undatus fruit" or "H. undatus fruit extract" or "the extract" refer to a fraction of the liquid isolated from the fleshy, edible part of the H. undatus (Pitahaya) fruit. The fraction is obtained by heating the pulp-free juice obtained after milling the fruit flesh and removing solid debris, separating solid particles produced during the heating and collecting the resulting clear liquid. The extract is characterized by emission of blue fluorescence when absorbing light having a wavelength of between 340nm to 395 nm with a peak of the emission at about 380nm. The extract is visible as blue to a human naked eye when placed on white paper without optical brighteners and illuminated at a wavelength range of 360nm - 370nm. As used herein, the terms "pulp-free juice" or "pulp-free fruit juice" refer to the juice of H. undatus fruit obtained by separating the edible fruit flesh from the pericarp, milling the fruit flesh to obtain slurry and separating solids from the slurry, typically by centrifugation, with no heating.

As used herein, the term "light source comprising UV light" refers to any light source comprising radiation at the UV range of from about 200nm to about 400nm, particularly from about 340nm to 395nm. According to some embodiments, the light source comprising UV radiation is sunlight. According to other embodiments, the light source comprising UV radiation is artificial light.

As used herein, the term "preservative" as used herein refers to a substance that is added to a composition of the invention to prevent contamination of H. undatus fruit extract by microbial growth and/or decomposition of the extract and/or its components by undesirable chemical changes subsequent to the microbial contamination.

The term "stabilizer" as used herein refers to a substance that is added to a composition of the invention to prevent or slow down physical and/or chemical changes to the composition and/or its component, particularly during storage. As is known in the art, a single compound can act both as a preservative and as a stabilizer.

As used herein, the term "visual perception" refers to the interpretation of the surrounding environment by processing information that is contained in visible light. The resulting perception is also known as eyesight, sight, or vision.

The term "cosmetic composition" as used herein refers to a composition suitable for topical application, particularly topical application onto skin in mammals.

According to certain embodiments, the composition has a cosmetically beneficial effect upon the skin as described herein.

Hylocereus Undatus (known by several synonyms including "Dragon fruit", "Pitaya" and Pitahaya") is a climbing vine cactus species which has received worldwide recognition as an ornamental plant and as a fruit crop. It is stabled as a crop inter alia in Australia, China, Israel, Taiwan and Vietnam. The fruit is a nutritious fruit with a variety of uses. The edible part of the fruit, the pulp, constitutes 70-80% of the ripe fruit. The fruit is known as having medicinal properties, including preventing colon cancer and diabetes, neutralizing toxic substances such as heavy metals, reducing cholesterol and high blood pressure, controlling bleeding and promoting dental health. The red-fleshed varieties are rich in antioxidants (Gunasena, H. P. M., et al. "Dragon Fruit Hylocereus undatus (Haw.) Britton and Rose." Underutilized fruit trees in Sri Lanka. New Delhi, World Agroforesty Centre (2007): 110-142).

Betalains are pigments known to be present in the dragon fruit red pericarp (skin) or within the flesh of red fruit. There are two categories of betalains: betacyanins, having reddish to violet color and betaxanthins with yellow to orange color. Betacyanins are known to emit light in the violet wavelength of about 400 nm. Betaxanthins exhibit spectra with excitation maxima between 463 nm and 474 nm (i.e. absorption in the blue light range) and emission maxima between 509 nm and 512 nm (i.e. in the green range).

To the contrary, the H. undatus fruit extract of the present invention absorbs light with a wavelength of between about 340 nm to about 395 nm that results in blue fluorescence emission (from about 430 nm to about 470 nm). Without wishing to be bound by any specific theory or mechanisms of action, the fluorescence of the extract of the invention cannot be attributed to the presence of betacyanins and/or betaxanthins. According to certain exemplary embodiments, the extract is devoid of betacyanins, betaxanthins or a combination thereof. According to some embodiments, the extract is devoid of betacyanins. According to other embodiments, extract is devoid of betaxanthins.

The present disclosure further shows that the absorbance and fluorescence profile of the H. undatus fruit extract of the present invention is different from that of the raw pulp-free fruit juice. As exemplified herein below, blue fluorescence is emitted by H. undatus fruit extract upon excitation with an extended wavelength range of from about 340 to 395 compared to such blue fluorescence which is emitted by the pulp-free fruit juice only upon excitation with about 360 nm.

Moreover, the blue fluorescence of the extract was significantly higher compared to that of the pulp-free fruit juice (Figure 1A-B). Addition of preservative and/or stabilizer to the extract did not significantly affect its blue fluorescence (Figure 1C).

Therapeutic and cosmetic use of H. undatus fruit extract has been previously described based, inter alia, on the showing that the extract is effective in slowing the proliferation of normal human fibroblast and/or keratinocyte cells in culture (US 2009/0264291). Slowing cell proliferation has a beneficial effect in preventing skin aging and maintaining the cell resources. As described therein, cosmetic application of the extract relies upon its reversible anti-proliferative activity. In the long term, inhibition of cell proliferation prolongs the life span of the skin and in the short term provides means for complete maturation of the cells. In both cases, the extract affects intrinsic activity of the cells to which it is applied, and continuous administration is required to achieve the desired effects of the anti-proliferative activity.

The blue light fluorescence of the extract of the invention has enormous potential for use in cosmetic products. The blue hue, coming in contact with the natural yellowish color of the skin, changes the appearance of the skin to be whiter. Furthermore, the general appearance of the skin and in particular the skin tone appears as more even. This phenomenon is distinct from the long term effect of H. undatus fruit extract on the natural luminescence of the skin, and lasts as long as the skin is covered by the H. undatus extract or a composition comprising same. The long term effect requires application of the extract onto the skin at least once daily for at least couple of weeks.

Advantageously, the extract does not confer any visually distinct color to the skin. The extract can thus be used as a "color free" makeup, significantly improving the skin appearance while keeping its natural appearance, with no "mask-like" look.

The extract of the invention or a composition comprising same can be used in a non-color cosmetic, e.g., a skin care or transparent or translucent cosmetic which is intended to confer little or no color to the skin after application. Alternatively, the extract or a composition comprising same can be added to a color cosmetic intended to mimic and improve the color of human skin, including, but not limited to a foundation, a blush, and a concealer, BB cream, CC cream.

According to one aspect, the present disclosure provides a method for reducing the visual perception of skin imperfection, the method comprises applying onto an imperfect skin area of a subject H. undatus fruit extract or a composition comprising same in an amount sufficient to provide thin layer cover of the imperfect skin area, thereby reducing the imperfection visuality of said skin area when exposed to UV radiation and/or sunlight.

According to certain embodiments, the method further comprises a step of identifying a human subject in need thereof.

According to certain exemplary embodiments, the present disclosure provides a method for reducing the visual perception of skin imperfection, the method comprises:
(a) identifying a human subject in need of reducing the visual perception of skin imperfection; and
(b) applying onto an imperfect skin area of the subject H. undatus fruit extract or a composition comprising same in an amount sufficient to provide thin-layer coverage of the imperfect skin area, thereby reducing the visibility of the imperfection of said skin area when exposed to UV light and/or sunlight.

According to certain embodiments, the subject is a human subject having a natural skin undertone selected from the group consisting of yellow, yellow-to-olive and olive.

According to some embodiments, identifying a human subject in need thereof is based on information received from the subject indicating said subject need for reducing the visual perception of skin imperfection. According to some embodiments, the information is received from answers of the subject to a questionnaire.

Designing and analyzing a questionnaire for identified a subject in need of reducing the visual perception of skin imperfection is well within the skills of a person with knowledge in the art. A questionnaire typically includes general questions about the subject gender, age and general health; questions regarding use of cosmetic, dermatological or therapeutic products; and request for information regarding area of skin imperfection. For identifying a subject in need thereof, the questionnaire is handed to a subject expressing interest before the treatment. The answers to the questionnaire are analyzed by a trained person who can design the application of the extract or a composition comprising same according to the subject needs.

According to yet additional embodiments, identifying a human subject in need thereof is based on professional assessment of the subject skin, particularly facial skin.

According to certain embodiments, the skin imperfection is selected from the group consisting of skin discolorations, open pores, mild scars, blotchiness and any combination thereof. Each possibility represents a separate embodiment of the present invention. It is to be explicitly understood that the term "skin discoloration" is meant herein in its broadest scope, and includes changes in the skin tone which may be the result of normal as well as of pathological process, for example skin pigmentation, acne, skin inflammation, use of certain drugs (such as minocycline and birth control pills), endocrine diseases such as Addison disease, hemochromatosis (iron overload), sun exposure, pregnancy, certain fungal infections (such as tinea versicolor); Pityriasis alba and vitiligo.

According to certain exemplary embodiments, the extract of the invention or a composition comprising same is used for reducing the appearance of dark shadows or lines on the skin.

According to other exemplary embodiments, the extract of the disclosure or a composition comprising same is used for improving the appearance and evening of the skin tone.

The extract of the present disclosure can be used alone as the primary component, for the purpose of evening skin tone, to disguise discolorations, dark shadows and under-eye circles on the skin.

The H. undatus fruit extract according to the teachings of the present disclosure can be incorporated into any kind of vehicle that is normally used in cosmetic compositions for facial care or care of any other part of exposed skin. For example, the extract can be added to solutions, colloidal dispersions, emulsions (oil-in-water or water-in-oil), suspensions, powders, creams, lotions, gels, foams, mousses, sprays. Methodology for formulation of different vehicle types is well known in the art, and can be found for example in Remington's The Science and Practice of Pharmacy, 19th Edition, Volume II.

According to some embodiments, the extract is used in a skin-toned powder color cosmetic, such as a face powder or body powder, or a powder blush. According to other embodiments, the extract can be used as part of a liquid, solid or semisolid color cosmetic, such as a liquid, cream, gel, or stick-type foundation, concealer, or blush. The compositions of this type are not brightly colored, but rather mimic the skin's natural color, and thus permit the most natural, advantageous and recognizable replenishment of the skin's youth look.

In the case of the use of the extract of the present disclosure in a skin-toned color cosmetic, the extract will confer substantially no color to the product. In a color cosmetic, the extract will be typically combined with other pigments or dyes. The additional color components can be either organic or inorganic. Examples of useful inorganic pigments include iron oxides (yellow, red, brown or black), ferric ammonium ferrocyanide (blue), manganese violet, ultramarine blue, chrome oxide (green), talc, lecithin modified talc, zeolite, kaolin, lecithin modified kaolin, titanium dioxide (white) and mixtures thereof. Other useful pigments are pearlescent pigments including mica, bismuth oxychloride and treated micas, such as titanated micas and lecithin modified micas.

Organic pigments include natural colorants and synthetic monomeric and polymeric colorants. Exemplary are phthalocyanine blue and green pigment, diarylide yellow and orange pigments, and azo-type red and yellow pigments such as toluidine red, litho red, naphthol red and brown pigments. Also useful are lakes, which are pigments, formed by the precipitation and absorption of organic dyes on an insoluble base, such as alumina, barium, or calcium hydrates. Polymeric colorants include nylon powder, polyethylene, and polyesters. The polyesters can include linear, thermoplastic, crystalline or amorphous materials produced using one or more diols and one or more dicarboxylic acids copolymerized with colorants. An exemplary list of cosmetically acceptable colorants can be found in the International Cosmetic Ingredient Dictionary and Handbook, 7th Edition, CTFA, 1997, pp. 1628-1630. In the color cosmetics of the present invention, colorants (not including the fluorescent extract of the invention) will normally constitute from about 0.1 % to about 30% by weight of the composition, the amounts varying depending upon the color desired.

In an alternate embodiment, the extract of the invention is employed in a non-color cosmetic, such as a transparent or translucent product.

The amount of the extract of the present disclosure within a cosmetic composition may be varied depending upon the intensity of the fluorescence, the purpose of the use and the type of skin to which the composition is applied. According to certain embodiments, the amount can be from about 0.01% to about 100%; According to other embodiments, the amount used will be between about 0.1 % up to about 50% or about 0.5% to about 50%. According to certain exemplary embodiments, the amount is from 0.5% to 5.0%. According to certain currently exemplary embodiments, the amount of H. undatus fruit extract within a cosmetic composition is 1.0%.

According to certain embodiments, the cosmetic composition further comprises a cosmetically or a dermatology acceptable diluent, excipient or carrier. According to some embodiments, the cosmetically acceptable carrier is selected from the group consisting of a liposome, a micelle structure, a microcapsule, and a combination thereof. Each possibility represents a separate embodiment of the present disclosure.

According to some embodiments, the cosmetic composition further comprises an acceptable additive. According to certain embodiments, the additive is selected from the group consisting of: stabilizers, preservatives pigments, pH buffers, emollients, fats, emulsifiers, co-emulsifiers, hydrophilic gelling agents, lipophilic gelling agents, solvents, fragrances, fillers, hydrophilic filters, lipophilic filters, dyestuffs, neutralizers, penetration-enhancing agents, polymers and any combination thereof. Each possibility represents a separate embodiment of the present disclosure.

The quantities of the various additives are those conventionally used in cosmetic and dermatological preparations as is known to a person skilled in the art. According to certain exemplary embodiments, the cosmetic composition comprising the H. undatus fruit extract further comprises a preservative and/or stabilizer. According to some embodiments, the preservative/stabilizer is selected from the group consisting of sodium metabisulfite, phenoxyethanol, benzyl alcohol, benzoic acid, its salts and esters, sorbic acid and its salts, parabens and their salts, triclosan, imidazolidinyl urea, phenoxyethanol, DMDM hydantoin, diazolidinyl urea and chlorphenesin. Each possibility represents a separate embodiment of the present disclosure.

According to additional embodiments, the composition comprising the extract of the invention further comprises additional skin care treatment active ingredient, including, but no limited to, active ingredients for improving or eradicating age spots, keratoses and wrinkles, analgesics, anesthetics, anti-acne agents, antibacterial agents, anti-yeast agents, antifungal agents, antiviral agents, anti-dermatitis agents, antipruritic agents, anti-inflammatory agents, anti-hyperkeratolytic agents, anti-dry skin agents, antiperspirants, anti-psoriatic agents, anti-seborrheic agents, anti-aging agents, anti-wrinkle agents, sunscreen agents, antihistamine agents, skin lightening agents, depigmenting agents, wound-healing agents, vitamins, corticosteroids and self-tanning agents. The amount of active agent to be used in any given formulation is readily determined in accordance with its usual dosage.

The mode of application of the compositions of the invention will depend upon the final intended use. In a color cosmetic/makeup product, the extract-containing composition will normally be applied on an as-needed basis, to the face, or optionally the body, as part of the user's daily makeup routine. As a non-color cosmetic, the composition can be applied daily, with or without makeup, simply to replenish the skin's natural appearance and to cause unadorned skin to appear healthier and without flaws. It may also be applied to particular trouble spots, such as dark under-eye shadows, or spots of uneven skin colors in order to reduce their visual appearance. The amount of product applied will also vary depending upon the final end use, and the appearance intended to be achieved, as long as a thin layer cover is achieved.

According to certain exemplary embodiments, a cosmetic composition comprising from about 1 % to about 5 % H. undatus fruit extract is applied onto the imperfect skin area at an amount of from 0.1 g to 5.0 g. According to certain exemplary embodiments, the composition comprises 1 % H. undatus fruit extract and is applied onto the imperfect skin area at an amount of from about 0.5 g to about 1.0 g.

According to additional aspects, the present disclosure provides a cosmetic composition comprising H. undatus fruit extract, wherein the composition is formulated as a makeup composition for the eyelashes, the eyebrows or the skin. Each possibility represents a separate embodiment of the present disclosure.

According to certain embodiments, the makeup composition is in a form selected from the group consisting of eye shadow, powder, lipstick, foundation, mascara, blush, and eyeliner.

According to some embodiments, the makeup composition is in a form selected from the group consisting of solid, pasty, anhydrous or aqueous form.

According to certain exemplary embodiments, the makeup composition form is selected from the group consisting of oil-in-water emulsion, water-in-oil emulsion, nonionic vesicular dispersion and suspension.

According to certain exemplary embodiments, the methods of the present disclosure employ the makeup composition described herein.

The following examples are presented in order to more fully illustrate some embodiments of the disclosure.

### EXAMPLES

### Example 1 : Production of H. undatus Fruit extract

Ripe Hylocereus Undatus fruit were washed, and the flesh was separated from the rind. The flesh was then milled (with food processor) until homogeneous, and the resulting slurry was centrifuged (15-60 min at at least 1800 g). The precipitate was discarded and the supernatant (pulp-free fruit juice) was collected.

The supernatant was heated to 100-120 °C for 0.5-2h. After heating, the resulting mixture was centrifuged (15-60 min at at least 1800 g). The precipitate was discarded and the supernatant was filtered through membrane filters, typically up to 0.22 micron, to obtain the H. undatus fruit extract.

Optionally, preservatives and stabilizers suitable for the relevant final application are added.

### Example 2: Fluorescence of H. undatus fruit extract

Unexpectedly, it was discovered that the H. undatus fruit extract produced as described hereinabove emits blue light upon radiation with UV light (358nm). Therefore, the fluorescent potential of this extract using a quantitative method enabling the screen of a wide spectrum of wavelength was examined using a fluorometer (FL920 Edinburgh Inc, UK). The fluorescence of the extract was compared to that of H. undatus pulp-free fruit juice. As described hereinabove, the pulp-free fruit juice is the liquid obtained after removal of insoluble solids from the milled slurry of the fruit flesh (before heating and further processing to obtain the extract of the invention). In addition, the effect of the addition of a preservative and a stabilizer to the composition was also examined: Phenoxyethanol (0.9%) and sodium metabisulfite (0.2%), respectively.

Excitation radiation was applied at intervals of 10 nm starting from 250 nm up to 600nm. Emission was measured every 3 nm. The measurements were taken using undiluted samples (100% extracts) in a 4-sided quartz cuvette suitable for fluorescence measurements. Comparing the emission data obtained for the three preparations showed a clear difference between the pulp-free fruit juice and the extract of the invention (Figure 1A and IB, respectively). While excitation at the range of 340-390 nm resulted in emission peaks at high intensity for the H. undatus fruit extract in the blue range only (430-470), the pulp-free juice showed significantly lower emission peaks and over the blue (450-460) and green range (515-535). The addition of Phenoxyethanol and sodium metabisulfite (MBS) to the H. undatus fruit extract resulted in some shift in the excitation wavelength, but preserved the high intensity peak in the blue range (445-465 nm).

### Example 3: Exemplary formulation

Table 1 below is an example formulation of cream containing 1% H. undatus fruit extract.

**Table 1 : Cream Formulation**

| **INCI/chemical name** | **Tradename** | **% in formula** |
|---|---|---|
| Water | Water | 84.35 |
| Butylene glycol | 1,3-butanediol | 4.00 |
| Dipropylene glycol | Dipropylene glycol | 1.00 |
| Hexylene glycol | Hexylene glycol | 1.00 |
| Polysorbate 20 | Tween 20 | 1.00 |
| Hydrogenated polydecene | Nexbase | 1.50 |
| Cyclomethicone | SF005Z | 4.00 |
| ***Hyclocereus fruit extract*** | | ***1.00*** |
| Carbomer | Carbopol 940 | 0.80 |
| Triethanolamine | Triethanolamine | 0.70 |
| Phenoxyethanol | Phenoxyethanol | 0.40 |
| Methyl paraben | Nipagin M | 0.15 |
| EDTA | EDTA | 0.10 |
| TOTAL | | 100.00 |

### Example 4: in vivo efficacy assessment of cream with 1% H. undatus fruit extract

A cream containing H. undatus fruit extract obtained as described in Example 1 above was used for preliminary assessment of the in vivo efficacy of the H. undatus fruit extract (the cream formulation presented in Table 1). One healthy volunteer participated in the assay. Pictures were taken under full-spectrum light and effect was assessed using image analysis from VISIA-CR®. First picture of the volunteer face (Figure 2A) was taken before application of the cream. Cream was then applied to form a thin layer covering the face. The second picture (Figure 2B) was taken 1 hour after the cream was applied. As is clear from comparing the two pictures, the cream containing the H. undatus fruit extract gives a perception of even skin tone, significantly reducing visuality of skin open pores and also of skin spots.

## Claims

1. A use of *Hylocereus undatus* fruit extract as light activated fluorescent colorant of skin for reducing the visual perception of skin imperfection, the use comprises applying onto an imperfect skin area of a human subject in need thereof *Hylocereus undatus* fruit extract or a composition comprising same in an amount sufficient to provide thin layer cover of the imperfect skin area, thereby reducing the visual perception of imperfection of said skin area when exposed to a light source comprising ultraviolet (UV) light, wherein the *H. undatus* fruit extract is produced by a process comprising the steps of:
a. separating the edible fruit flesh from the pericarp;
b. milling the fruit flesh to obtain a slurry;
c. separating insoluble solids from the slurry to obtain a pulp-free juice;
d. heating the pulp-free juice; and
e. separating insoluble solids from the heated pulp-free juice to obtain a liquid extract.

2. The use of claim 1,
wherein the skin undertone color of the human subject is selected from the group consisting of yellow, yellow to olive and olive undertone.

3. The use of claim 1 or 2;
wherein the skin imperfection is selected from the group consisting of skin open pores, skin discoloration, mild scars, blotchiness, and any combination thereof or uneven skin tone.

4. The use of one of claims 1 to 3,
wherein the *H. undatus* fruit extract is **characterized by** fluorescence emission at the blue range upon excitation over wavelength range of from 340 nm to 395 nm.

5. The use of one of claims 1 to 4,
wherein the *H. undatus* fruit extract is **characterized by** a fluorescence emission having a peak at a wavelength range of from 460 nm to 470 nm upon excitation with light having a wavelength of 370 nm to 390 nm.

6. The use of one of claims 1 to 5,
wherein the composition comprising the *H. undatus* fruit extract is a cosmetic composition further comprising a cosmetically acceptable stabilizer, preservative or a combination thereof.

7. The use of claim 6,
wherein the cosmetic composition further comprises a cosmetically acceptable diluent, carrier or excipient.

8. The use of claim 6 or claim 7,
wherein the cosmetic composition is formulated in a form selected from the group consisting of powders, solutions, creams, emulsions, suspensions, dispersions, ointments and sticks.

9. The use of one of claims 6 to 8,
wherein the cosmetic composition is in a form selected from the group consisting of a foundation, a concealer, a facial powder and a body powder, or wherein the cosmetic composition is in a form selected from the group consisting of a blush, an highlighter, an eyeshadow, an eyeliner and a contouring preparation.

10. The use of one of claims 6 to 9,
wherein the cosmetic composition is non-color cosmetic composition; or wherein the cosmetic composition is color cosmetic composition.

11. The use of one of claims 6 to 10,
wherein the cosmetic composition further comprises at least one cosmetically acceptable colorant.

12. The use of any one of claims 6 to 11,
wherein the cosmetic composition is a make-up composition.

13. The use of any one of claims 6 to 12, wherein the cosmetic composition comprises from 0.5% to 50% of the *H. undatus* fruit extract.

14. The use of one of claims 1 to 13,
wherein the reduction in the visual perception of skin imperfection lasts for up to 12 hours from the time of applying the *H. undatus* fruit extract or the composition comprising same.

## Patentansprüche

1. Verwendung von Hylocereus-Undatus-Fruchtextrakt als lichtaktivierter fluoreszierender Hautfarbstoff zur Verringerung der visuellen Wahrnehmung von Hautunvollkommenheiten, wobei die Verwendung das Auftragen von Hylocereus-Undatus-Fruchtextrakt oder einer diesen enthaltenden Zusammensetzung auf einen unvollkommenen Hautbereich eines menschlichen Subjekts, das dessen bedarf, in einer Menge aufweist, die ausreicht, um den unvollkommenen Hautbereich mit einer dünnen Schicht zu bedecken, wodurch die visuelle Wahrnehmung der Unvollkommenheit des Hautbereichs verringert wird, wenn dieser einer Lichtquelle ausgesetzt wird, die ultraviolettes (UV) Licht aufweist, wobei der Hylocereus-Undatus-Fruchtextrakt durch ein Verfahren hergestellt ist, das die folgenden Schritte aufweist:
a. Abtrennen des essbaren Fruchtfleisches vom Perikarp;
b. Mahlen des Fruchtfleisches, um eine Aufschlämmung zu erhalten;
c. Abtrennen unlöslicher Feststoffe aus der Aufschlämmung, um einen faserfreien Saft zu erhalten;
d. Erhitzen des faserfreien Saftes; und
e. Abtrennen der unlöslichen Feststoffe aus dem erhitzten, faserfreien Saft, um einen flüssigen Extrakt zu erhalten.

2. Verwendung nach Anspruch 1,
wobei die Hautuntertonfarbe des menschlichen Subjekts ausgewählt ist aus der Gruppe bestehend aus gelbem, gelbem bis olivem und olivem Unterton.

3. Verwendung nach Anspruch 1 oder 2,
wobei die Hautunvollkommenheit ausgewählt ist aus der Gruppe bestehend aus offenen Poren der Haut, Hautverfärbung, leichten Narben, Flecken und jeder Kombination davon oder ungleichmäßigem Hautton.

4. Verwendung nach einem der Ansprüche 1 bis 3,
wobei der Hylocereus-Undatus-Fruchtextrakt durch Fluoreszenzemission im blauen Bereich bei Anregung über einen Wellenlängenbereich von 340 nm bis 395 nm gekennzeichnet ist.

5. Verwendung nach einem der Ansprüche 1 bis 4,
wobei der Hylocereus-Undatus-Fruchtextrakt durch eine Fluoreszenzemission mit einem Peak bei einem Wellenlängenbereich von 460 nm bis 470 nm bei Anregung mit Licht einer Wellenlänge von 370 nm bis 390 nm gekennzeichnet ist.

6. Verwendung nach einem der Ansprüche 1 bis 5,
wobei die Zusammensetzung, die den Hylocereus-Undatus-Fruchtextrakt aufweist, eine kosmetische Zusammensetzung ist, die ferner einen kosmetisch akzeptablen Stabilisator, ein Konservierungsmittel oder eine Kombination davon aufweist.

7. Verwendung nach Anspruch 6,
wobei die kosmetische Zusammensetzung ferner ein kosmetisch akzeptables Verdünnungsmittel, einen Träger oder einen Hilfsstoff aufweist.

8. Verwendung nach Anspruch 6 oder Anspruch 7,
wobei die kosmetische Zusammensetzung in einer Form ausgebildet ist, die ausgewählt ist aus der Gruppe bestehend aus Pulvern, Lösungen, Cremes, Emulsionen, Suspensionen, Dispersionen, Salben und Stiften.

9. Verwendung nach einem der Ansprüche 6 bis 8,
wobei die kosmetische Zusammensetzung in einer Form vorliegt, die ausgewählt ist aus der Gruppe bestehend aus einer Foundation, einem Concealer, einem Gesichtspuder und einem Körperpuder, oder wobei die kosmetische Zusammensetzung in einer Form vorliegt, die ausgewählt ist aus der Gruppe bestehend aus einem Rouge, einem Highlighter, einem Lidschatten, einem Eyeliner und einer Konturierungszubereitung.

10. Verwendung nach einem der Ansprüche 6 bis 9,
wobei die kosmetische Zusammensetzung eine nicht-farbige kosmetische Zusammensetzung ist; oder
worin die kosmetische Zusammensetzung eine farbige kosmetische Zusammensetzung ist.

11. Verwendung nach einem der Ansprüche 6 bis 10,
wobei die kosmetische Zusammensetzung ferner mindestens einen kosmetisch akzeptablen Farbstoff aufweist.

12. Verwendung nach einem der Ansprüche 6 bis 11,
wobei die kosmetische Zusammensetzung eine Make-up-Zusammensetzung ist.

13. Verwendung nach einem der Ansprüche 6 bis 12, wobei die kosmetische Zusammensetzung 0,5% bis 50% des Hylocereus-Undatus-Fruchtextrakts enthält.

14. Verwendung nach einem der Ansprüche 1 bis 13,
wobei die Verringerung der visuellen Wahrnehmung von Hautunreinheiten bis zu 12 Stunden ab dem Zeitpunkt der Anwendung des Hylocereu-Undatus-Fruchtextrakts oder der diesen enthaltenden Zusammensetzung anhält.

## Revendications

1. Utilisation d'extrait de fruit d'hylocereus undatus comme colorant fluorescent de peau activé par la lumière pour réduire la perception visuelle d'une imperfection de peau, l'utilisation comprenant d'appliquer, sur une zone de peau imparfaite d'un sujet humain qui en a besoin, un extrait de fruit d'hylocereus undatus ou une composition le comprenant dans une quantité suffisante pour fournir une couverture à couche mince de la zone de peau imparfaite, réduisant ainsi la perception visuelle de l'imperfection de ladite zone de peau quand elle est exposée à une source de lumière comprenant une lumière à ultraviolets (UV), dans laquelle l'extrait de fruit d'hylocereus undatus est produit par un procédé comprenant les étapes consistant à :
a. séparer la chair du fruit comestible du péricarpe ;
b. broyer la chair du fruit pour obtenir une bouillie ;
c. séparer les solides insolubles de la bouillie pour obtenir un jus exempt de pulpe ;
d. chauffer le jus exempt de pulpe ; et
e. séparer les solides insolubles du jus exempt de pulpe chauffé pour obtenir un extrait liquide.

2. Utilisation selon la revendication 1,
dans laquelle la couleur du sous-teint de peau du sujet humain est sélectionnée parmi le groupe constitué de : sous-teint jaune, jaune à olive et olive.

3. Utilisation selon la revendication 1 ou 2,
dans laquelle l'imperfection de peau est sélectionnée parmi le groupe constitué de : pores ouverts de la peau, décoloration de la peau, cicatrices légères, couperose, et une combinaison quelconque parmi ces imperfections ou un teint de peau irrégulier.

4. Utilisation selon l'une des revendications 1 à 3,
dans laquelle l'extrait de fruit d'hylocereus undatus est **caractérisé par** une émission de fluorescence au niveau de la gamme de bleu lors d'une excitation sur une plage de longueur d'onde allant de 340 nm à 395 nm.

5. Utilisation selon l'une des revendications 1 à 4,
dans laquelle l'extrait de fruit d'hylocereus undatus est **caractérisé par** une émission de fluorescence ayant un pic au niveau d'une plage de longueur d'onde allant de 460 nm à 470 nm lors d'une excitation avec une lumière ayant une longueur d'onde de 370 nm à 390 nm.

6. Utilisation selon l'une des revendications 1 à 5,
dans laquelle la composition comprenant l'extrait de fruit d'hylocereus undatus est une composition cosmétique comprenant en outre un stabilisateur ou un conservateur cosmétiquement acceptables, ou une combinaison de ceux-ci.

7. Utilisation selon la revendication 6,
dans laquelle la composition cosmétique comprend en outre un diluant, un support ou un excipient cosmétiquement acceptables.

8. Utilisation selon la revendication 6 ou 7,
dans laquelle la composition cosmétique est formulée dans une forme sélectionnée parmi le groupe constitué de : poudres, solutions, crèmes, émulsions, suspensions, dispersions, pommades et bâtons.

9. Utilisation selon l'une des revendications 6 à 8,
dans laquelle la composition cosmétique est dans une forme sélectionnée parmi le groupe constitué de : fond de teint, correcteur, poudre pour le visage et poudre pour le corps ; ou dans laquelle la composition cosmétique est dans une forme sélectionnée parmi le groupe constitué de : fard à joues, enlumineur, fard à paupières, eye-liner et préparation de contouring.

10. Utilisation selon l'une des revendications 6 à 9,
dans laquelle la composition cosmétique est une composition cosmétique non colorée ; ou
dans laquelle la composition cosmétique est une composition cosmétique colorée.

11. Utilisation selon l'une des revendications 6 à 10,
dans laquelle la composition cosmétique comprend en outre au moins un colorant cosmétiquement acceptable.

12. Utilisation selon l'une quelconque des revendications 6 à 11,
dans laquelle la composition cosmétique est une composition de maquillage.

13. Utilisation selon l'une quelconque des revendications 6 à 12,
dans laquelle la composition cosmétique comprend de 0,5 % à 50 % d'extrait de fruit d'hylocereus undatus.

14. Utilisation selon l'une des revendications 1 à 13,
dans laquelle la réduction dans la perception visuelle de l'imperfection de peau dure jusqu'à 12 heures à partir du moment de l'application de l'extrait de fruit d'hylocereus undatus ou de la composition le comprenant.
